Europäisches Patentamt

European Patent Office

Office européen des brevets

(19)

(11) Publication number: **0 495 488 A2**

## EUROPEAN PATENT APPLICATION

(12)

(21) Application number: **92100620.1**

(22) Date of filing: **15.01.92**

(51) Int. Cl.5: **A61B 17/16, A61B 17/58**

(30) Priority: **15.01.91 US 642982**

(43) Date of publication of application:
**22.07.92 Bulletin 92/30**

(84) Designated Contracting States:
**AT BE CH DE DK ES FR GB GR IT LI LU NL PT SE**

(71) Applicant: **Pfizer Hospital Products Group, Inc.**
**235 East 42nd Street**
**New York New York 10017(US)**
Applicant: **Krag, Martin H.**
**25 Crooked Creek Road**

**Colchester, VT 05446(US)**

(72) Inventor: **Krag, Martin H.**
**25 Crooked Creek Road**
**Colchester, VT 05446(US)**
Inventor: **Crombie, John S.**
**12 Delmar Place**
**Irvington, NJ 07111(US)**

(74) Representative: **Lederer, Franz, Dr. et al**
**Lederer, Keller & Riederer, Patentanwälte,**
**Lucile-Grahn-Strasse 22**
**W-8000 München 80(DE)**

(54) **Surgical drill guide.**

(57) A drill guide for use in surgical procedures, especially in implanting spinal fixation devices is provided, having an extended handle portion (1) in articulated relationship to a radiolucent drill guide holder (5). A drill guide tube (6) is mounted in the drill guide holder, and has an inwardly chamfered end (7) for gaining a positive footing in the area to be drilled.

FIG. 2

FIG. 3

EP 0 495 488 A2

Field of the Invention

The present invention relates to drill guides used in surgical procedures, particularly involving spinal fixation.

Background of the Invention

Medical treatment of trauma to the spine includes the implantation of an internal fixation device to fuse the spine across the injured section. One example of such a spinal fixation device is known as a Vermont Spinal Fixator ("VSF"). Design and testing of VSF devices is discussed at length in M. Krag, et al., "An Internal Fixator for Posterior Application to Short Segments of the Thoracic, Lumbar, or Lumbosacral Spine", Clinical Orthopaedics and Related Research 203:75-98 (February 1986), which is incorporated herein by reference.

A VSF generally includes two brace members, one attached on each side of the spinal column, and each brace having a pair of screws mounted on either side of the injured location, and a longitudinal member connecting the pair of screws to form a rigid structure. Other similar fixation devices which use screws for attachment to the pedicle also exist.

According to the standard medical procedure, it is preferred to mount the screws into the pedicle portions of the vertebrae. This procedure is known from extensive clinical practice to be reliable, and is further considered trustworthy based on experiences with other procedures involving operation on the pedicle, such as biopsies and bone grafts. The pedicles are pairs of short, thick protrusions having roughly circular cross-sections that extend from the anterior body portion to the posterior and transverse portion of each vertebra. The cross-sectional area of the pedicle decreases at a middle region, so that the pedicle is roughly the shape of a cylinder with a constriction toward the center of its length.

Drilling for mounting the VSF screws is done perpendicularly through the circular cross-section of the pedicle. A drill guide is used in the procedure to control the location and orientation of the drill bit. Precise alignment of the bit is necessary to ensure that the hole is drilled down the very center of the pedicle. It is crucial that such alignment be achieved because of the narrowing portion of the pedicle toward its middle. If the drilling deviates from its central path, there is a danger that the bit or subsequently the screw will break through the side of the pedicle.

The position of the drill bit is controlled by channeling the bit through a tube, which is part of a drill guide. In order to determine the exact position of the tube beyond the tolerances and capabilities of the naked eye, radiography is used to ascertain the relationship of the tube to patterns which are detectable in radiographic images of the bone matter. Specifically, when the x-ray beam is oriented along the pedicle axis, approximately perpendicular to the pedicle cross-section, the pedicle will appear as a ring. The end of the tube that contacts the bone is then located so as to appear in the radiograph as within the pedicle ring. The tube is oriented to be parallel to the x-ray beam so that only its circular cross-section is visible and not its side walls. With the drill bit guided by the tube positioned in this way, correct placement of the drill bit in the pedicle is provided.

Prior art drill guides employed in the foregoing procedure have a number of shortcomings. Although prior guides disclose the basic design of a guide tube mounted on a handle, it is difficult to maintain adequate control over the positioning of the drill bit using such implements. First, the overall structure of prior art guides is not conducive to attaining a stable, leveraged positioning of the guide tube at the drilling location. Second, prior guides are incapable of adjustment between the handle and the guide tube, which compromises adaptability to varying conditions and further contributes to problems with stability. Third, the prior art devices fail to address the desirability of minimizing the interference which the instruments produce in the radiographic image, particularly due to interference with the image of the bony matter caused by metal or other components close to the tube. Finally, even when proper alignment is achieved, the guide tubes of prior art devices are prone to slippage at the end of the tube which contacts the vertebra.

Summary of the Invention

It is therefore an object of this invention to provide a drill guide which is of a shape and size that facilitates stability and control of the instrument during drilling procedures.

It is a further object of the invention to provide such an instrument which is capable of adjustment through a range of positions to further facilitate ease of use and control of the instrument.

It is another object of the invention to minimize the interference which the drill guide produces in radiographic images used during the drilling procedure.

It is yet another object of the invention to provide a guide tube which is not susceptible to slippage once positioned at the drilling surface.

These and other objects which would be apparent to one skilled in the art are achieved by the present invention, which comprises a drill guide having an extended handle portion in articulated relation with a radiolucent guide tube holder. A guide tube mounted in the guide tube holder has an end with an inward chamfer.

The extended handle and articulation of the instrument permit optimal adjustment and positioning during the drilling procedure. Specifically, the present invention can be firmly positioned against a patient's body to achieve a high level of leverage and stability. The articulated relationship of the components makes possible minor adjustments to the position of the guide tube while the instrument remains firmly in place. Significant advantages are consequently derived in the ease and effectiveness of use of the instrument.

The guide tube is perpendicularly fixed to the guide tube holder, which in turn is attached in articulated relationship to the handle of the drill guide. The guide tube holder is constructed of a radiolucent material and shaped to prevent interference in the radiographic image in the vicinity of the guide tube.

Finally, chamfering the end of the guide tube only on its inner surface as taught by the present invention prevents slippage of the tube when placed on sloping surfaces.

## Brief Description of the Drawings

Fig. 1 is a top view of the drill guide according to the present invention;

Fig. 2 is a side view of the drill guide of Fig. 1;

Fig. 3 is a perspective view of the chamfered end of the guide tube according to the present invention;

Fig. 4 is a cross-sectional view of the chamfered end of the guide tube taken along line 4-4 of FIG. 3;

Fig. 5 is a perspective view of a vertebrae with the guide tube of the present invention positioned thereon;

Fig. 6 is a detailed view of a prior art drill guide tube positioned on a vertebrae; and

Fig. 7 is a detailed view of a drill guide tube according to the present invention positioned on a vertebra.

## Description of the Preferred Embodiments

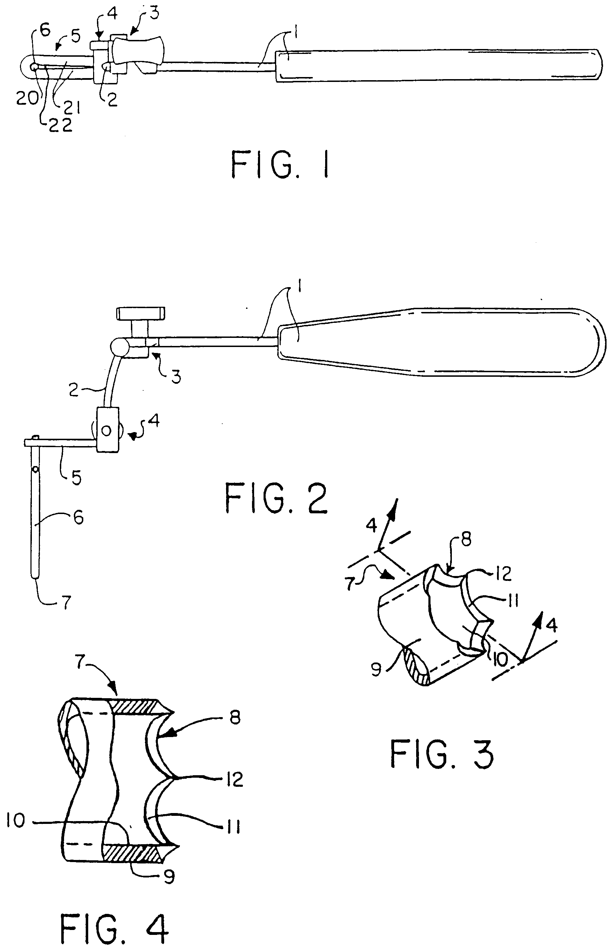

Figs. 1 and 2 show a preferred configuration of the present invention. Articulation of the drill guide is provided by screw clamp 3 which adjustably connects handle 1 to rod member 2. This arrangement provides an articulated connecting means for joining the drill guide tube 6 to the handle 1 such that the angle between those parts may be selectively adjusted. The handle 1 is of an extended length to allow the user to brace it against a surface external to the vertebra being operated on and space the user's hand away from the x-ray beam. It has been found particularly useful to brace the handle against the body of the patient. The ability to brace the handle 1 in this manner improves the reliability of the drill guide and eases the task of the user during operating procedures by providing enhanced ability to keep the guide absolutely steady once the proper position is attained. Moreover, it is possible to maintain such a strongly braced position without obstructing the operating area.

The connection at clamp 3 provides for adjustment of the angle between handle 1 and drill guide tube 6. Rod member 2 extends to a second screw clamp 4 provides for attachment and removal of drill guide tube holder 5. Drill guide tube holder 5 extends from screw clamp 4, and drill guide tube 6 is mounted in guide tube holder 5. Guide tube 6 serves to direct the drill bit to the proper drilling location and maintain its placement there. It also protects soft tissue around the drilling area from the rotating drill elements.

As shown in Fig. 1, guide tube holder 5 is preferably an elongated member having a hole 20 at a first end for holding guide tube 6, and a slot 22 running from said hole down the length of the member to the second end of the member, such that the slot divides the member between the hole and the second end into two side portions 21. These side portions are spread apart to allow for insertion of the guide tube 6 into the hole at the first end, and are then urged together and clamped in a closed position at screw clamp 4 to grip guide tube 6 within holder 5. This design of holder 5 allows for securing varying sizes of tubes and facilitates exchanging the tubes for one another. Different diameter tubes are required depending on the size screw being implanted. Over sizing of the tube is to be avoided because of a resulting loss of positional control of the drill bit within the tube.

Guide tube holder 5 is preferably of a radiolucent material to avoid causing interference in radiographic images. The configuration of guide tube holder 5 also serves to space the metal mass of the remainder of the drill guide components away from the critical area of the guide tube, thus further reducing radiographic interference. Radiolucent materials suitable for use with the invention include plastic and fiberglass.

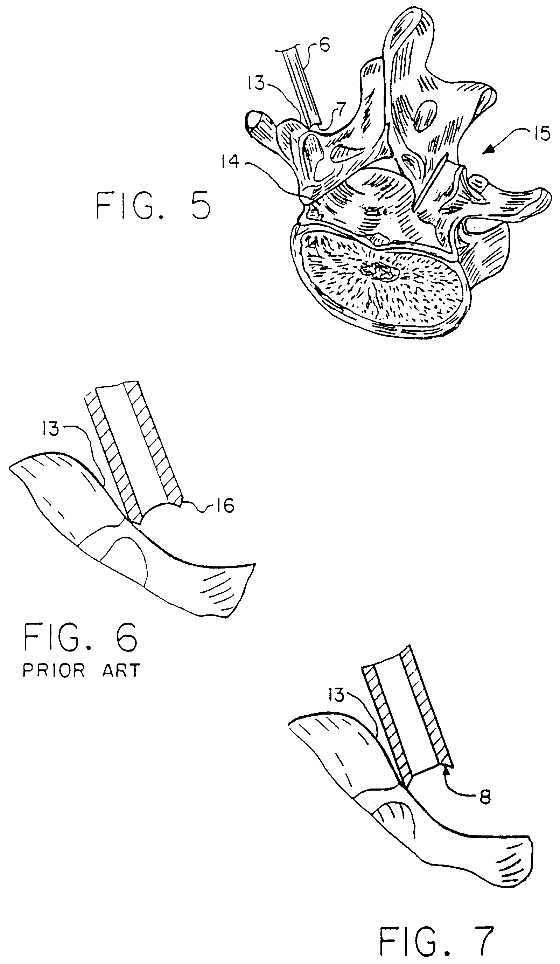

Figs. 3 and 4 show enlarged views of end 7 of guide tube 6. End 7 has an internal chamfer 8 so that the outer wall 9 of guide tube 6 extends slightly further than inner wall 10. This chamfer 8 allows for tube 6 to be effectively anchored in the area to be drilled as shown in FIG. 7. Prior art devices having external chamfers are inferior for such purposes because they tend to slip from positions on sloping surfaces. Chamfer 8 is further characterized by concave recesses 11 around the circumference of end 7, which form sharp edges ending in points 12 at the junctures between recesses 11. This serrated configuration enhances the anchoring of guide tube 6 in the area of the drilling location.

Fig. 5 shows the drill guide tube 6 of the present invention positioned at a surface 13 overlying pedicle portion 14 of a vertebra 15. The advantages of the internally-chamfered end 7 of guide tube 6 are demonstrated in Fig. 7. Fig. 6 shows a prior art type of guide tube having an external chamfer 16. When this external chamfer 16 is placed on a sloping surface 13 overlying pedicle 14 it can glance off. Thus, prior art external chamfers lack stability and tend to slip when pressure is exerted in an attempt to anchor a guide tube. The use of an internal chamfer 8 in the present invention, as shown in Fig. 7, enables the user to firmly implant the guide tube even on a steeply sloping surface.

While it is apparent that the invention herein disclosed is well calculated to fulfill the objectives stated above, it will be appreciated that numerous modifications and embodiments may be devised by those skilled in the art. It is intended that the appended claims cover all such modifications and embodiments as fall within the true spirit and scope of the present invention.

**Claims**

1. A drill guide, comprising:

    a tubular member having an inner and an outer wall, a first end portion, and a second end portion for anchoring said tubular member on a surface in the area of a location to be drilled, said second end portion having an internally chamfered surface extending from the outer wall at said second end of said tubular member, toward said first end portion of said tubular member, to the inner wall of said tubular member.

2. The drill guide of claim 1 wherein said chamfered end portion has a plurality of concave recesses, and wherein junctures between adjacent concave recesses form sharp edges ending in points at said second end.

3. A drill guide for use in surgical procedures in incisions in a patient, comprising:

    a tubular member having first and second end portions and inner and outer walls, for guiding a drill bit to a desired location for drilling; and

    articulated connecting means for joining said tubular member to an extended handle, wherein said connecting means allows for adjusting the orientation of said tubular member with respect to said extended handle.

4. The drill guide of claim 3, wherein said articulated connecting means comprises a radiolucent drill guide holder for spacing other drill guide components away from said tubular member and avoiding interference in radiographic images taken while using the drill guide, said holder connected to the first end portion of said tubular member.

5. The drill guide of claim 3, wherein the second end portion of said tubular member has an internally chamfered surface extending from the outer wall of said tubular member at said second end toward the first end of said tubular member, to the inner wall of said tubular member, for anchoring said tubular member on a surface in the area of a location to be drilled.

6. The drill guide of claim 5, wherein said chamfered end portion has a plurality of concave recesses, and wherein junctures between adjacent concave recesses form sharp points at said second end.

7. The drill guide of claim 3, wherein said extended handle is of sufficient length to stabilize said tubular member by bracing said handle against the patients body a sufficient distance from the incision to avoid obstructing the surgical procedure.

8. The drill guide of claim 3, wherein said connecting means comprises a rod member joined at a first end to said handle, and a drill guide holder joined at a first end to a second end of said rod member and at a second end to said first end of said tubular member, and wherein said rod member is angularly adjustable with respect to said handle.

9. The drill guide of claim 8, wherein said drill guide holder is radiolucent, spacing other non-radiolucent drill guide components away from said tubular member and avoiding interference in radiographic images taken while using the drill guide, and wherein the second end portion of said tubular member has an internally chamfered surface extending from the outer wall of said tubular member at said second end toward the first end of said tubular member, to the inner wall of said tubular member, for anchoring said tubular member on a surface in the area of a location to be drilled.

10. A drill guide, comprising:

a tubular member for guiding a drill bit to a desired location for drilling, said tubular member having first and second end portions and inner and outer walls, said second end portion having an internally chamfered surface extending from the outer wall of said tubular member at said second end, toward the first end of said tubular member, to the inner wall of said tubular member, said chamfered end portion having a plurality of concave recesses, and wherein junctures between adjacent concave recesses form sharp points at said second end;

an extended handle of sufficient length to stabilize said tubular member by bracing said handle against an external surface spaced a distance away from the location of drilling; and

articulated connecting means for joining said tubular member to said extended handle, said connecting means comprising

a rod member having first and second ends, joined at the first end to said handle, and

a radiolucent drill guide holder having first and second ends, joined at the first end to the second end of said rod member and joined at the second end to said first end of said tubular member, said rod member being positionally adjustable with respect to said handle and said drill guide holder, for facilitating adjustment of and controlling the position of said tubular member with respect to said handle.

11. A drill guide tube holder for securing a drill guide tube to a drill guide handle, comprising an elongated member of a radiolucent material having a first end portion defining a hole for receiving a drill guide tube and side portions extending from said first end portion, said side portions defining a slot there between and said hole opening into said slot, whereby said side portions may be spread apart to widen said hole to facilitate insertion of a drill guide tube and urged together for biased engagement of the tube.

12. The drill guide tube holder of claim 11, wherein said radiolucent material is selected from the group consisting of plastic and fiberglass.

13. A method of guiding a drill for implanting a fixation device in a spine, comprising:

positioning a tubular member, having a first and second end portions and a handle structure, for guiding a drill bit through a pedicle of a vertebra;

aligning said tubular member concentrically with said pedicle by radiographically imaging said pedicle and tubular member and adjusting the position of said tubular member so that the radiographic image of said tubular member is concentrically disposed within a circular image representing said pedicle, and so that the images of the pedicle and tubular member are unobscured by the handle structure in the radiographic image;

anchoring an end portion of said tubular member on a bony surface overlying said pedicle while maintaining said tubular member in said alignment, wherein said tubular member has an inner wall and an outer wall and a chamfered surface at said second end portion extending from said outer wall into said inner wall toward the first end of said tubular member; and

drilling said pedicle by guiding said drill bit through said tubular member into said pedicle.

14. The method of claim 13, wherein said tubular member is connected to the handle structure by articulated connecting means, and said method further comprising bracing said handle structure against a surface external to the spine for stabilizing said tubular member in said alignment.

15. The method of claim 14, wherein said articulated connecting means comprises a radiolucent drill guide holder for securing said tubular member to said articulated connecting means.

5

FIG. 1

FIG. 2

FIG. 3

FIG. 4

FIG. 5

FIG. 6
PRIOR ART

FIG. 7